# EUROPEAN PATENT APPLICATION

(11) **EP 2 617 380 A1**
(43) Date of publication of application: **24.07.2013**
(21) Application number: 13151667.6
(22) Date of filing: 17.01.2013
(51) Int. Cl.: A61B 18/20, A61N 5/06

(54) **A system of remote controlling a medical laser generator unit with a portable computing device**

(30) Priority: 20.01.2012 US 201213355483
(71) Applicant: Villa, Cristiano, Euclid, OH 44123 (US)
(72) Inventor: Villa, Cristiano, Euclid, OH 44123 (US)
(74) Representative: Hocking, Adrian Niall

(57) **Abstract**

In reference to FIG. 1, the system of remotely controlling the medical laser generator unit (1) through a portable computing device (23) such as a smart phone or a tablet personal computer allows the user to control the properties of the medical laser with a downloadable software application. The portable computing device (23) and the medical laser generator unit (1) communicate with each other through a network connection (32), which can be either a hard-wired link or a wireless link. The properties of the medical laser that can be controlled by the software application include emission power, pulse structure, and treatment duration. The software application is also able to retrieve feedback data from the patient during a medical procedure. The feedback data includes tissue color, tissue temperature, and a laser plume signature. The software application allows the user to access each individual patient's medical information so that the user can better perform the medical procedure.

## Description

### FIELD OF THE INVENTION

The present invention relates generally to a system of delivering a medical laser through a laser generator unit and remotely controlling the laser generator unit through a portable computing device such as a smart phone or a tablet personal computer.

### BACKGROUND OF THE INVENTION

Traditionally, a large laser generator unit is built on or as a self-contained cart. The placement of the cart during a medical procedure is relatively inflexible in relation to a patient and a medical practitioner. The medical practitioner must move away from the patient and towards the cart to monitor and reset the laser emission parameters. A different space management problem arises from a small laser generator unit, which has a relatively small control system. The medical practitioner must move the medical laser pen away from the patient during the medical procedure and focus on the small laser generator unit to monitor and reset the laser emission parameters and to recharge its battery. Therefore, the objective of the present invention is to provide a system to remotely control a medical laser generator unit with a portable computing device, which allows more flexibility in managing the space between the medical practitioner, the patient, and the medical laser generator unit. If the medical practitioner is relatively ambidextrous, then he/she may operate the portable computing device in one hand and use the medical laser pen to complete the medical procedure in the other hand.

### BRIEF DESCRIPTION OF THE DRAWINGS

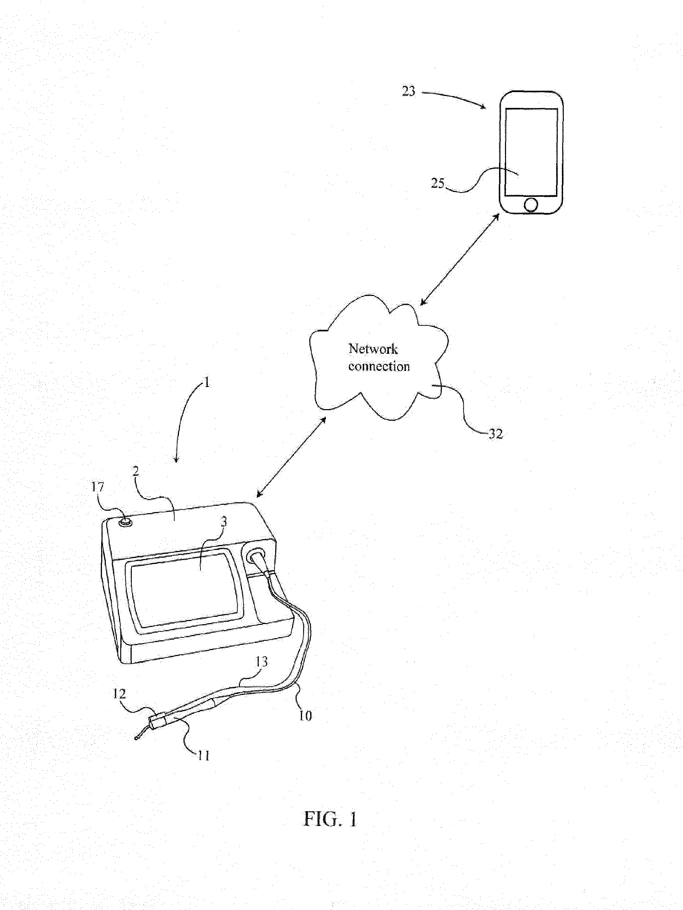

FIG. **1** is a schematic view illustrating the system between the medical laser generator unit and the portable computing device.

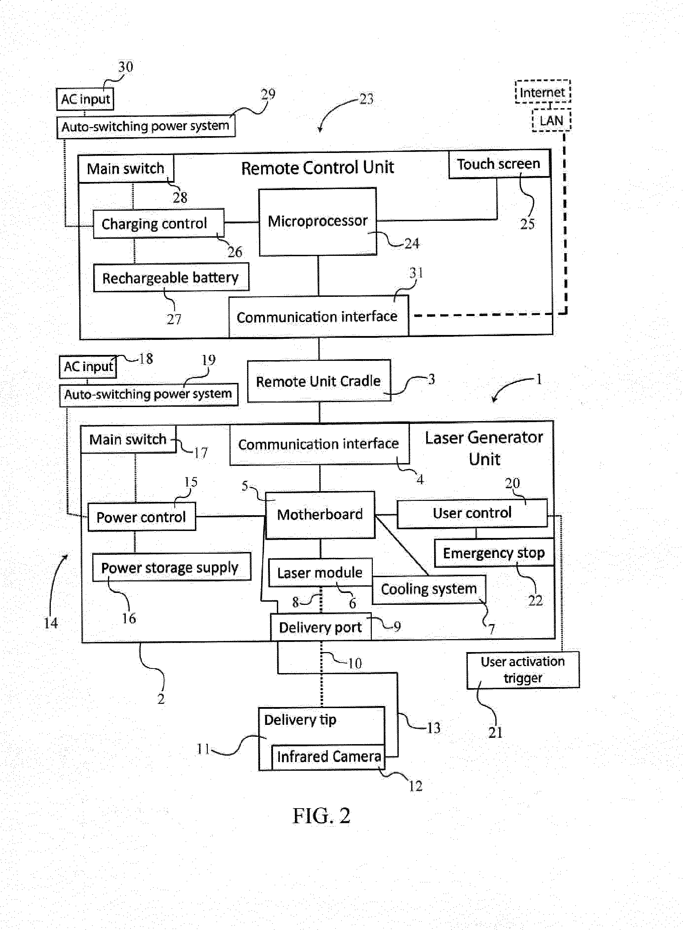

FIG. **2** is a schematic view of the medical laser generator unit, the portable computing device, and both of their components.

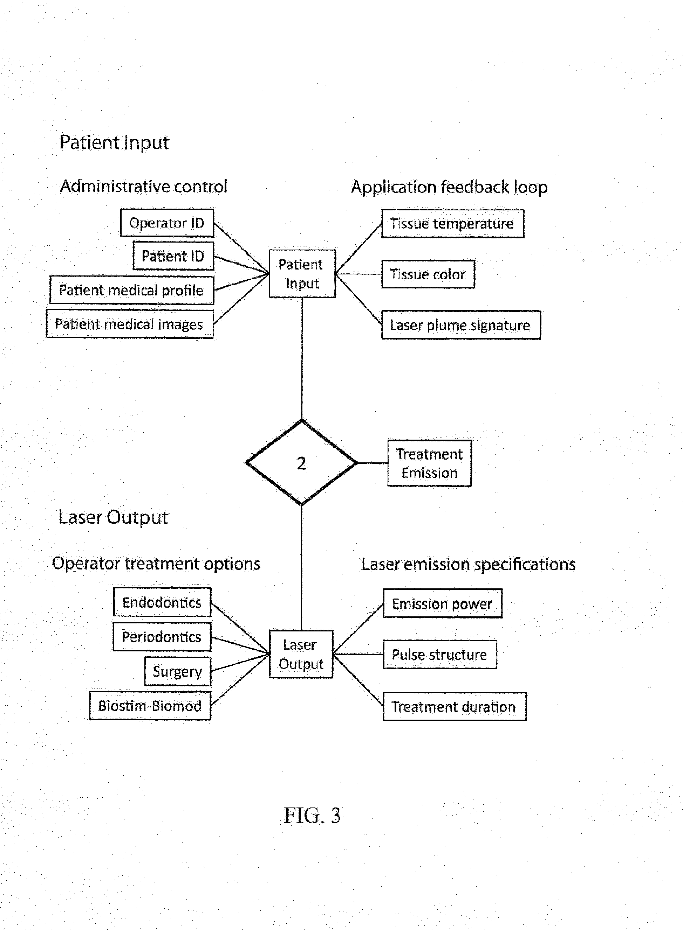

FIG. **3** is a software schematic for the portable computing device, which depicts the major components of the remote control software.

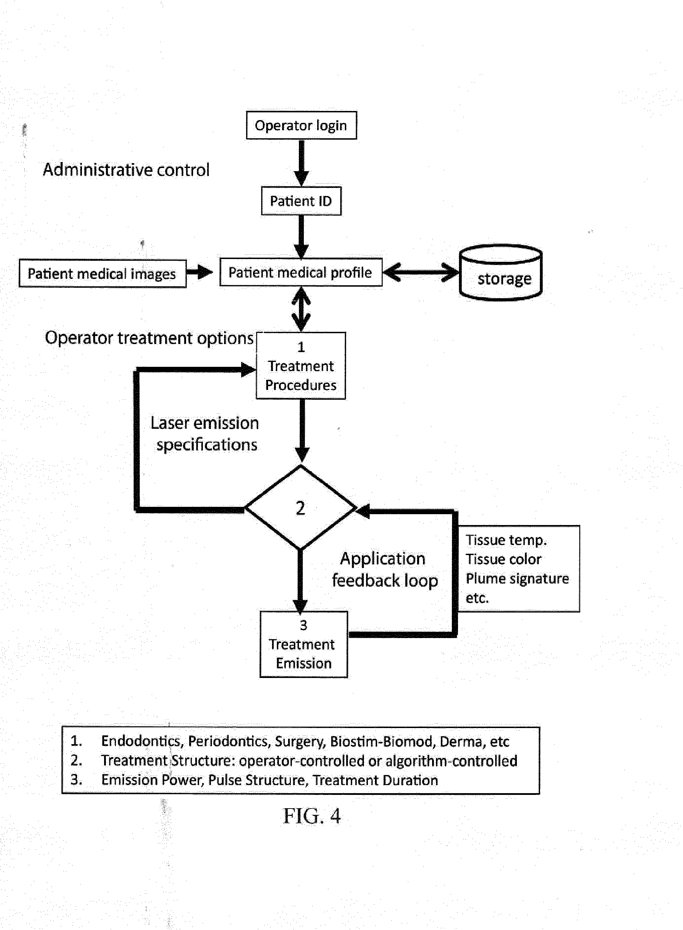

FIG. **4** is a software schematic illustrating the general flow of the remote control software.

### DETAIL DESCRIPTIONS OF THE INVENTION

All illustrations of the drawings are for the purpose of describing selected versions of the present invention and are not intended to limit the scope of the present invention.

The present invention is a medical laser generator unit 1 that allows a user to remotely control its functions with a portable computing device **23** such as a smart phone or tablet computer. The medical laser generator unit **1** comprises an enclosure **2,** a remote control cradle **3,** a communication interface **4,** a motherboard **5,** a power supply system **14,** a laser module **6,** an internal fiber optic cable **8,** a delivery port **9,** an external fiber optic cable **10,** a delivery tip **11,** a user control **20,** an user activation trigger **21,** and an emergency stop **22.** The enclosure **2** is the casing that houses and protects the electronic components of the medical laser generator unit **1.** The enclosure **2** also provides a base to attach the other components of the medical laser generator unit **1.** The motherboard **5** and the communication interface **4** are located inside of the enclosure **2.** The motherboard **5** interprets the instructions that are sent by the portable computing device **23** to the medical laser generator unit **1.** The motherboard **5** also implements the control logic that is used to manage the other electronic components. The motherboard **5** is able to communicate with the portable computing device **23** with the communication interface **4,** which is electronically connected to the motherboard **5.** The communication interface **4** can either provide a hard wire link to the portable computing device **23,** such as Universal Serial Bus (USB) or Recommend Standard 232 (RS232), or provide a wireless link to the portable computing device **23,** such as WiFi or Bluetooth. The remote control cradle **3** allows portable computing device **23** to physically attach to the medical laser generator. The remote control cradle **3** is electronically connected to the communication interface **4,** which creates a hard wire link between the portable computing device **23** and the medical laser generator unit **1** once the portable computing device **23** is attached to the remote control cradle **3.**

The components that are used to emit the medical laser are the laser module **6,** an internal fiber optic cable **8,** a delivery port **9,** an external fiber optic cable **10,** and a delivery tip **11.** The laser module **6** produces the medical laser either with a semiconductor (diode) or by laser pumping (gas and crystal). The laser module **6** comprises a cooling system **7** because the laser module **6** generates heat while producing the medical laser. Both the laser module **6** and the cooling system **7** are electronically connected to the motherboard **5,** which allows the motherboard **5** to manage the laser module **6** and the cooling system **7.** The internal fiber optic cable **8** optically connects the laser module **6** to the delivery port **9,** which allows the medical laser to travel from the laser module **6** to the delivery port **9.** The laser module **6** is optically connected to the delivery port **9** because the internal fiber optic cable **8** uses total internal reflection to guide the medical laser to the delivery port **9.** The delivery port **9** is positioned on the enclosure **2** and traversing into the enclosure **2.** The delivery port **9** is a universal receptacle that can engage different kinds of delivery tips **11,** which are used for a variety of medical procedures. The delivery tip **11** is a physical apparatus that emits the medical laser and allows the user to handle and point the medical laser. The external fiber optic cable **10** optically connects the delivery tip **11** to the delivery port **9,** which allows the medical laser to travel from the delivery port **9** to the delivery tip **11.** The external fiber optic cable **10** also uses total internal reflection to guide the medical laser to the delivery tip **11.** The external fiber optic cable **10** is flexible so that the user can easily maneuver the delivery tip **11.** In another embodiment of the present invention, the internal fiber optic cable **8** and the external fiber optic cable **10** can be replaced by a series of the mirrors that reflect the medical laser along a guided path. The delivery tip **11** is also equipped with an infrared camera **12** that are able to measure the tissue temperature, the tissue color, and the laser plume signature during a medical procedure. The infrared camera **12** is electronically connected to the motherboard **5** by a data cable **13,** which allows the motherboard **5** to retrieve the tissue temperature, the tissue color, and the laser plume signature as feedback loop data. The data cable **13** traverses through the delivery port **9** to electronically connect to the motherboard **5.**

The power supply system **14** provides electrical power to all of the electronic components of the medical laser generator unit **1.** The power supply system **14** comprises a power control **15,** a power storage supply **16,** a main switch **17,** an alternative current input (AC input **18**), and an auto-switching power system **19.** The AC input **18** can be plugged into any standardize outlet in order to power the medical laser generator unit **1.** The AC input **18** is electrically connected to the auto-switching power system **19,** which is used to convert the voltage from a standardized outlet into a voltage that better accommodates the medical laser generator unit **1.** The auto-switching power supply is basically a step-down transformer. The auto-switching power system **19** is electrically connected to the power control **15** through the enclosure **2.** The power control **15** regulates how much electrical power from the AC input **18** is used to charge the power storage supply **16** and how much electrical power from the AC input **18** is used to power the other electrical components of the medical laser generator unit **1.** If the power storage supply **16** is not completely charged, then the power control **15** diverts power from the AC input **18** into the power storage supply **16.** If the power storage supply **16** is completely charged, then the power control **15** does not divert any power from the AC input **18** to the power storage supply **16.** The power storage supply **16** can be either a battery or a capacitor and provides power to the other electronic components if the AC input **18** is accidently disconnected from the standardized outlet. Once the power control **15** does not detect any incoming power from the AC input **18,** the power control **15** automatically starts to withdraw power from the power storage supply **16** for the other electronic components, which allows the user to continue the medical procedure without any interruptions. The power control **15** is electronically connected to the motherboard **5,** which allows the power control **15** to pass on the power from either the AC input **18** or the power storage supply **16.** The main switch **17** is positioned on the enclosure **2** and is electrically connected to the power control **15** through the enclosure **2.** The main switch **17** allows the user to turn on or off the power to the medical laser generator. When the main switch **17** is activated, the main switch **17** sends an analog signal that indicates whether the power should be turned on or off. The power control **15** interprets the analog signal from the main switch **17** and effectively turns the power on or off.

When the power for the medical generator unit is turned on, the user is able to activate the medical laser with two components: the user activation trigger **21** and the emergency stop **22.** The user activation trigger **21** is a physical means that allows the user to turn the medical laser on or off during a medical procedure with either their hand, their foot, or their voice. In the preferred embodiment of the present invention, the user activation trigger **21** is either a finger switch or a foot pedal that turns the medical laser on and off. The emergency stop **22** is a physical means, such as a button, that allows the user to completely shut down the laser module **6** so that the medical laser cannot be turned on. The emergency stop **22** is used to prevent any harm to the patient if the user activation trigger **21** or any other part of the medical laser generator start starts to malfunction. The user activation trigger **21** is located outside of the enclosure **2,** and the emergency stop **22** is positioned on the enclosure **2.** Both the user activation trigger **21** and the emergency stop **22** are electrically connected to the user control **20** through the enclosure **2.** The user control **20** is used to convert the analog signal transmitted by either the user activation trigger **21** or the emergency stop **22** into a digital signal. The user control **20** is electronically connected to the motherboard **5,** which allows the user control **20** to send the digital signal from either the user activation trigger **21** or the emergency stop **22** to the motherboard **5.**

Different kinds of portable computing devices **23** usually comprise similar components. Those components include a microprocessor **24,** a touch screen **25,** a charging control **26,** a rechargeable battery **27,** a device main switch **28,** a device auto-switching power system **29,** a device AC input **30,** and a device communication interface **31.** The microprocessor **24** is used to manage the other electronic components of the portable computing device **23.** The microprocessor **24** also executes a remote control software, which allows the user to operate the medical laser generator unit 1 from the portable computing device **23.** The touch screen **25** is electronically connected to the microprocessor **24** and is used to display a graphic user interface, which allows the user to interact with the remote control software. The device communication interface **31** is electronically connected to the motherboard **5,** which allows the device communication interface **31** to transmit instructions, that are created by the remote control software, to the medical laser generator unit **1.** Similar to the medical laser generator unit 1, the device communication interface **31** should be able to communicate with the medical generator unit through a hard-wire link or a wireless link, such as USB, RS232, WiFi, and Bluetooth.

The portable computing device **23** uses components that are similar to the medical laser generator unit 1 to power its electronic components. The device AC input **30** draws electrical power from a standardized outlet, and the device auto-switching power system **29** converts the electrical power into a usable form for the portable computing device **23.** The device AC input **30** is electrically connected to the device auto-switching power system **29,** and the device auto-switching power system **29** is electrically connected to the charging control **26.** The rechargeable battery **27** is a portable power source that is also electrically connected to the charging control **26.** The charging control **26** is electronically connected to the motherboard **5** and regulates the power from both the AC input **18** and the rechargeable battery **27** to each other and to the other electronic components of the portable computing device **23.** The main switch **17** is electrically connected to charging control **26** and allows the user to turn on or off the power from the AC input **18** and the rechargeable battery **27.**

The medical laser generator unit **1** and the portable computing device **23** are able to communicate data along three different communication links: a first channel, a second channel, and a third channel. The first channel is a hard-wired link between the device communication interface **31** of the portable computing device **23** and the communication interface **4** of the medical laser generator unit **1,** which allows the microprocessor **24** and the motherboard **5** to communicate with each other. The second channel is a wireless link between the device communication interface **31** of the portable computing device **23** and the communication interface **4** of the medical laser generator unit **1,** which allows the microprocessor **24** and the motherboard **5** to communicate with each other. The first channel and the second channel are used by the portable computing device **23** and the medical laser generator unit **1** to transfer data and instructions that is required by the remote control software to operate the medical laser generator unit **1** from the portable computing device **23.** The first channel and the second channel can both be considered a network connection **32** that links the medical laser generator unit **1** to the portable computing device **23.** Thus, the network connection **32** can be either a software connection or a hardware connection. The network connection **32** allows the medical laser generator unit **1** and the portable computing device **23** to be an adequately working system. The third channel is hard-wired link between the delivery tip **11** and the motherboard **5** and is used to communicate the feedback loop data to the motherboard **5,** where the remote control software can retrieve the feedback loop data from either the first channel or the second channel.

The remote control software is executed by the microprocessor **24** of the portable computing device **23.** The remote control software has a number of components that are given before the microprocessor **24** begins the process to operate the medical laser generator from the portable computing device **23.** One given component is a patient database with a plurality of patient files and is used to store all information for individual patients. Each of the plurality of patient files contains medical information for each individual patient, which includes a patient medical profile and patient medical images. The patient medical profile outlines the medical history, the medication history, and medical procedure history for an individual patient as well as other pertinent information about the individual patient. The patient medical images are images of the individual patient's body that are used for clinical procedures to reveal, diagnose, or examine a medical disease afflicting the individual patient. Each of the plurality of the patient files has a patient identification, which differentiates each patient file from the plurality of patient files. Another given component is a treatment database, which is used to store a plurality of treatment templates. Each of the plurality of treatment templates is used to identify a particular kind of medical procedure, such as endodontics, periodontics, surgery, and biostim-biomod, which can be optimized by adjusting the properties of the medical laser. Each of the plurality of treatment templates also has a set of laser parameter preset values, which contain the numeric values that the properties of the medical laser should be adjusted to in order to obtain the best treatment results for the particular kind of medical procedure. Each of the plurality of treatment templates also has a template red spectrum wavelength, which is the ideal red spectrum wavelength of the medical laser for a particular kind of medical procedure. The patient database and the treatment database could be located within the portable computing device **23** and/or located on a server that multiple users can access from any portable computing device **23.** The portable computing device **23** connects to the server through a Local Area Network (LAN) and/or through the internet. Another given component is the graphic user interface that allows the user to interact with the remote control software and allows the user to continuously monitor the properties of the medical laser.

The process that is followed by the remote control software allows the user to control the medical laser generator unit 1 with the portable computing unit. The process begins by retrieving an operator identification, which only authorized personnel to access the remote control software because the remote control software allows the user to access confidential patient information. The process continues by retrieving the patient identification for the individual patient that is undergoing the medical procedure. The patient identification allows the remote control software to search through the plurality of patient files in order to find the patient medical profile and the patient medical images for the individual patient. The remote control software then retrieves the patient medical profile and the patient medical images from the patient database and displays the patient medical profile and the patient medical images to the user through the graphic user interface. Thus, the user is informed on the medical history of the individual patient before the user begins the medical procedure.

Once the initial setup for the medical procedure is done, the remote control software prompts the user to initiate the medical procedure. If the user agrees to begin the medical procedure, the remote control software will record the medical procedure to the patient medical profile, which allows the patient database to have the most up-to-date information on an individual patient. Next, the remote control software will want to adjust a plurality of laser parameters so that the medical laser is better suited for the medical procedure. The plurality of laser parameters includes a laser emission power setting, a pulse structure, and an emission duration setting. The laser emission power setting can be adjusted according to watts from zero to maximum. The pulse structure setting can be adjusted according to frequency in hertz, to time-on in milliseconds or microseconds, or to time-off in milliseconds or microseconds. The plurality of laser parameters can be adjusted with two different methods. For one method, the remote control software will prompt the user to manually adjust the laser emission power setting, the pulse structure setting, and the emission duration setting through the graphic user interface. For the other method, the remote control software will prompt the user to choose one of the plurality of treatment templates to adjust the plurality of laser parameters. Once the user chooses a treatment template, the remote control software will retrieve the laser parameter preset values for that particular treatment template from the treatment database. The laser parameter preset values will then be applied to the laser emission power setting, the pulse structure setting, and the emission duration setting.

Once the plurality of laser parameters have been set, the remote control software will send instructions to the medical laser generator unit **1** through either the first channel or the second channel. During the emission of the medical laser, the remote control software will begin to display each of the plurality of laser parameters and a main power control **15** on the graphic user interface. The main power control **15** allows the user to stop the emission of the medical laser with the graphic user interface. Also during the emission of the medical laser, the remote control software will retrieve feedback loop data from the delivery tip **11** through the third channel and through either the first channel or the second channel. The feedback loop data is collected in real-time and includes the tissue temperature, the tissue color, and the plume signature.

The remote control software implements a semi-automated process and an automated process to fine tune the plurality of laser parameters, which only occur if the user initially chooses to adjust the plurality of laser parameters with the laser parameter preset values. The semi-automated process begins by prompting the user to readjust the plurality of laser parameters with the patient medical images. When the user accepts, the remote control software will extract the chromophore content data from the patient medical images, which allows the remote control software to determine an image red spectrum wavelength for the patient medical images. The remote control software will then determine a set of laser parameter image values by comparing the template red spectrum wavelength to the image red spectrum wavelength. Finally, the remote control software will apply the set of laser parameter image values to the laser emission power setting, the pulse structure setting, and the emission duration setting. Similarly, the automated process begins by prompting the user to readjust the plurality of laser parameters with the feedback loop data. When the user accepts, the remote control software will extract the chromophore content data from the feedback loop data, which allows the remote control software to determine a feedback red spectrum wavelength for feedback loop data. The remote control software will then determine a set of laser parameter feedback values by comparing the template red spectrum wavelength to the feedback red spectrum wavelength and the image red spectrum wavelength. The remote control software will then apply the set of laser parameter image values to the laser emission power setting, the pulse structure setting, and the emission duration setting. After both the semi-automated process and the automated process are complete, the laser parameter feedback values and the laser parameter image values are converted into a new patient profile entry, which is added the patient medical profile.

Although the invention has been explained in relation to its preferred embodiment, it is to be understood that many other possible modifications and variations can be made without departing from the spirit and scope of the invention as hereinafter claimed.

## Claims

1. A medical laser generator unit (1) comprises,
an enclosure (2);
a remote control cradle (3);
a communication interface (4);
a motherboard (5);
a power supply system (14);
a laser module (6);
an internal fiber optic cable (8);
a delivery port (9);
an external fiber optic cable (10);
a data cable (13);
a delivery tip (11);
a user control (20);
a user activation trigger (21);
an emergency stop (22);
said delivery tip (11) comprises an infrared camera (12);
said laser module (6) comprises a cooling system (7); and
said motherboard (5), said communication interface (4), said laser module (6), said cooling system (7), and said user control (20) being located within said enclosure (2).

2. A medical laser generator unit (1) as claimed in claim 1 comprises,
said motherboard (5) being electronically connected to said laser module (6);
said cooling system (7) being electronically connected to said motherboard (5);
said delivery port (9) being positioned on said enclosure (2) and traversing into said enclosure (2);
said laser module (6) being optically connected to said delivery port (9) by said internal fiber optic cable (8);
said delivery tip (11) being optically connected to said delivery port (9) by said external fiber optic cable (10);
said infrared camera (12) being positioned on said delivery tip (11);
said infrared camera (12) being aligned to said delivery tip (11);
said infrared camera (12) being electronically connected to said motherboard (5) by said data cable (13); and
said data cable (13) traversing through said delivery port (9).

3. A medical laser generator unit (1) as claimed in claim 1 comprises,
said remote control cradle (3) being positioned on and connected to said enclosure (2);
said remote control cradle (3) being electronically connected to said communication interface (4); and
said communication interface (4) being electronically connected to said motherboard (5).

4. A medical laser generator unit (1) as claimed in claim 1 comprises,
said power supply system (14) comprises a power control (15), a power storage supply (16), a main switch (17), an alternating current (AC) input (18), and an auto-switch power system (19);
said power control (15) being electronically connected to said motherboard (5);
said main switch (17) being positioned on and connected to said enclosure (2);
said main switch (17) being electrically connected to said power control (15);
said AC input (18) traversing through said enclosure (2) and being electrically connected to said power control (15); and
said power storage supply (16) being electrically connected to said power control (15).

5. A medical laser generator unit (1) as claimed in claim 1 comprises,
said user control (20) being electronically connected to said motherboard (5);
said user activation trigger (21) being electrically connected to said user control (20);
said user activation trigger (21) being located outside of said enclosure (2);
said emergency stop (22) being positioned on and connected to said enclosure (2); and
said emergency stop (22) being electrically connected to said user control (20).

6. A method of operating a medical laser generator unit (1) with a portable computing device (23) by executing computer-executable instructions stored on a non-transitory computer-readable medium, the method comprises the steps of:
providing a patient database with a plurality of patient files, wherein each of said plurality of patient files has a patient medical profile and patient medical images;
providing a treatment database with a plurality of treatment templates, wherein each of said plurality of treatment templates has laser parameter preset values and a template red spectrum wavelength;
providing a patient identification for each of said plurality of patient files;
providing a graphic user interface;
retrieving an operator identification;
retrieving said patient identification;
searching through said plurality of patient files with said patient identification to find said patient medical profile and said patient medical images;
retrieving said patient medical profile and said patient medical images from said patient database;
displaying said patient medical profile and said patient medical images;
prompting to initiate a medical procedure and recording said medical procedure to said patient medical profile;
adjusting a plurality of laser parameters for said medical procedure, wherein said plurality of laser parameters includes a laser emission power setting, a pulse structure setting, and an emission duration setting;
sending instructions to emit a medical laser according to said plurality of laser parameters;
displaying said plurality of laser parameters and a main power control (15) on said graphic user interface; and
retrieving feedback loop data, wherein said feedback data includes tissue temperature, tissue color, and plume signature.

7. A method of operating a medical laser generator unit (1) with a portable computing device (23) by executing computer-executable instructions stored on a non-transitory computer-readable medium, the method as claimed in claim 6 comprises the steps of:
prompting to adjust said laser emission power setting, said pulse structure setting, and said emission duration setting through said graphic user interface.

8. A method of operating a medical laser generator unit (1) with a portable computing device (23) by executing computer-executable instructions stored on a non-transitory computer-readable medium, the method as claimed in claim 6 comprises the steps of:
prompting to choose from said plurality of treatment templates to adjust said plurality of laser parameters;
retrieving said laser parameter preset values from said treatment database;
and
applying said laser parameter preset values to said laser emission power setting, said pulse structure setting, and said emission duration setting.

9. A method of operating a medical laser generator unit (1) with a portable computing device (23) by executing computer-executable instructions stored on a non-transitory computer-readable medium, the method as claimed in claim 8 comprises the steps of:
prompting to readjust said laser emission power setting, said pulse structure setting, and said emission duration setting with said patient medical images;
extracting chromophore content data from said patient medical images;
determining an image red spectrum wavelength from said chromophore content data;
retrieving said template red spectrum wavelength from said treatment database;
determining laser parameter image values by comparing said template red spectrum wavelength to said image red spectrum wavelength; and
applying said laser parameter image values to said laser emission power setting, said pulse structure setting, and said emission duration setting.

10. A method of operating a medical laser generator unit (1) with a portable computing device (23) by executing computer-executable instructions stored on a non-transitory computer-readable medium, the method as claimed in claim 9 comprises the steps of:
prompting to readjust said laser emission power setting, said pulse structure setting, and said emission duration setting with said feedback loop data;
extracting chromophore content data from said feedback loop data;
determining a feedback red spectrum wavelength from said chromophore content data;
determining laser parameter feedback values by comparing said template red spectrum wavelength to said feedback red spectrum wavelength and said image red spectrum wavelength; and
applying said laser parameter feedback values to said laser emission power setting, said pulse structure setting, and said emission duration setting.

11. A method of operating a medical laser generator unit (1) with a portable computing device (23) by executing computer-executable instructions stored on a non-transitory computer-readable medium, the method as claimed in claim 10 comprises the steps of:
converting said laser parameter feedback values and said laser parameter image values into a new patient profile entry; and
adding said new patient profile entry in said patient medical profile.
